# EUROPÄISCHE PATENTSCHRIFT

(11) **EP 2 108 306 B1**
(45) Veröffentlichungstag und Bekanntmachung des Hinweises auf die Patenterteilung: **15.10.2014**
(21) Anmeldenummer: 09004958.6
(22) Anmeldetag: 03.04.2009
(51) Int. Cl.: A61B 1/04, G01B 11/00

(54) **Vorrichtung und Verfahren zur endoskopischen 3D-Datenerfassung**
Device and method for endoscopic 3D data creation
Dispositif et procédé de saisie de données 3D endoscopique

(30) Priorität: 11.04.2008 DE 102008018636
(43) Veröffentlichungstag der Anmeldung: 14.10.2009
(73) Patentinhaber: Storz Endoskop Produktions GmbH, 78532 Tuttlingen (DE)
(72) Erfinder: Krattiger, Beat, Dr., 8222 Beringen (CH)
(74) Vertreter: Fugmann, Winfried

(56) Entgegenhaltungen:
- DE-A1- 19 840 862
- DE-A1-102006 017 003
- DE-U1- 9 321 266
- US-A1- 2006 025 692
- US-A1- 2007 149 858
- US-B1- 6 293 911

## Beschreibung

Die vorliegende Erfindung betrifft eine Vorrichtung zur endoskopischen 3D-Datenerfassung nach dem Oberbegriff von Anspruch 1 sowie ein entsprechendes Verfahren.

Vorrichtungen und Verfahren zur endoskopischen 3D-Datenerfassung sind bekannt. So wird in US 20060055942 A1 ein Verfahren zur endoskopischen Distanzmessung beschrieben, das auf der parallaktischen Verschiebung auf das beobachtete Objekt projizierter Messpunkte beruht. Solche Verfahren sind relativ robust, die Genauigkeit hängt jedoch entscheidend von der Triangulationsbasis ab, die bei endoskopischen Anwendungen stets eng begrenzt ist. Darüber hinaus müssen diese Verfahren mit relativ wenigen Datenpunkten auskommen.

In DE 10 2006 017 003 A1 wird ein Endoskop zur Tiefenakquisition beschrieben, bei dem ein moduliertes Lichtsignal ausgesandt wird und die Modulationsparameter des empfangenen Lichtsignals zur Berechnung der Tiefendaten verwendet werden. Über einen als Strahlteiler verwendeten planen halbdurchlässigen Spiegel können zwei Bildsensoren Strahlung empfangen, wovon einer die zur Erzeugung von 3D-Daten nutzbaren Modulationsparameter aufnimmt, während der andere zur Aufnahme eines visuellen Bildes der endoskopischen Szene vorgesehen ist.

In US 2007/0149858 A1 ist ein Endoskop mit einer Fluoreszenzbeleuchtung offenbart, bei dem hinter einem Strahlteiler zwei Photodioden angeordnet sind, um aus dem Beleuchtungsstrahlengang rückgeführtes Fluoreszenzanregungs- und Fluoreszenzlicht zu messen und hierdurch mögliche Defekte des Beleuchtungsstrahlengangs zu detektieren. In US 2006/0025692 A1 wird eine Endoskopvorrichtung beschrieben, die ein optisches Element zur Trennung von Fluoreszenz- und reflektiertem Licht sowie zwei nachfolgend angeordnete CCDs umfasst, auf denen jeweils von einer Fokussierlinse ein Bild erzeugt wird; Bildsensoren mit unterschiedlichen aktiven Flächen sind dabei nicht vorgesehen.

In DE 198 40 862 A1 ist ein Fiber-Endoskop mit zwei Bildaufnahme-Einrichtungen beschrieben, die unterschiedliche optische Achsen aufweisen und wechselweise aktiviert werden können; hierdurch sind weniger Schwenkbewegungen erforderlich, um alle Bereiche eines Körperinnenraums zu erfassen. Gemäß DE 93 21 266 U1 umfasst ein optisches System mit einem variablen Abbildungsmaßstab zur Abbildung eines von einem Mikroskop erzeugten Bildes auf ein CCD einer Videokamera eine Linsenkombination, wobei ein zweites und drittes optisches Glied jeweils zur Variation des Abbildungsmaßstabs verschiebbar sind.

Es wäre häufig wünschenswert, auch weitere Arten von Bildsensoren einsetzen zu können, um beispielsweise IR- oder spektral selektierte bzw. aufgelöste Bilder aufzunehmen und mit den 3D-Daten gemeinsam darzustellen oder auszuwerten. Verschiedene Arten von Bildsensoren haben jedoch häufig unterschiedliche Formate bzw. Bilddiagonalen. Bei den im Stand der Technik bekannten Anordnungen kann dann jeweils nur das Bildfeld des kleinsten Sensors vollständig genutzt werden. Wird der Bildsensor mit der kleinsten aktiven Fläche vollständig genutzt, so bleibt bei einem Bildsensor mit einer größeren Fläche ein Teil der Fläche ungenutzt.

Es ist daher die Aufgabe der vorliegenden Erfindung, eine Vorrichtung und ein Verfahren zur endoskopischen 3D-Datenerfassung anzugeben, bei dem die aktiven Flächen von Bildsensoren auch unterschiedlicher Größen, Formate und/oder Seitenverhältnisse optimal genutzt werden können.

Diese Aufgabe wird durch eine Vorrichtung mit den Merkmalen des Anspruchs 1 bzw. durch ein Verfahren mit den Merkmalen des Anspruchs 14 gelöst.

Dadurch, dass mindestens eine Anpassungsoptik vorgesehen ist zur Anpassung der Gesichtsfelder der Bildsensoren, können die aktiven Flächen der Bildsensoren auch dann optimal genutzt werden, wenn diese unterschiedlich groß sind oder unterschiedliche Seitenverhältnisse aufweisen.

Eine kontinuierliche Modulation der Messstrahlung kann mit relativ einfachen elektronischen Mitteln erzeugt werden, und phasenempfindliche Festkörpersensoren sind einfach aufgebaut, leicht zu handhaben und kostengünstig verfügbar. Mit einer entsprechenden Modulationsfrequenz können Zeitverzögerungen, die Abstandsunterschieden im mm-Bereich bis über mehrere Meter hinaus entsprechen, einfach und sicher nachgewiesen werden. Durch die pixelaufgelöste Erfassung und Auswertung der Phaseninformationen ist die Erzeugung eines Bildes, das ortsaufgelöste Tiefen- bzw. Abstandsinformationen darstellt, möglich. Durch die Hinzunahme des Bildes eines beliebigen, auch ein unterschiedliches Format aufweisenden weiteren Bildsensors ist eine einfache und sichere Orientierung im 3D-Bild ebenso wie die Gewinnung weiterer, beispielsweise spektral selektierter bzw. aufgelöster Informationen möglich.

Eine erfindungsgemäße Vorrichtung umfasst Lichterzeugungsmittel zur Erzeugung mindestens einer modulierten Messstrahlung. Zur Erzeugung der Messstrahlung können dabei insbesondere lichtemittierende Dioden (LEDs), Superlumineszenzdioden, Laser, insbesondere Laserdioden oder Superkontinuum-Laser, oder andere entsprechend modulierbare Strahlungsquellen eingesetzt werden. Hierbei haben insbesondere Laserdioden den Vorteil einer einfachen Handhabung und sind kostengünstig, kompakt und leicht modulierbar. Dabei haben multimodale Laserdioden i. d. R. den Vorteil einer höheren Ausgangsleistung als monomodale Laserdioden.

Die Messstrahlung ist dabei beispielsweise sinusförmig modulierbar, ggf. über einem Grundniveau. Zur besseren Handhabung und effektiveren Kühlung können die Lichterzeugungsmittel in einem eigenen Gehäuse bzw. als separate Lichtquelle angeordnet sein.

Weiterhin umfasst eine erfindungsgemäße Vorrichtung Lichtweiterleitungsmittel zur Weiterleitung der Messstrahlung auf einen zu beobachtenden Bereich. Die Lichtweiterleitungsmittel können dabei insbesondere Mittel zur Einkopplung der von der Lichtquelle erzeugten Strahlung in einen Lichtleiter umfassen, sowie Lichtleiter zur Weiterleitung der Strahlung. So kann beispielsweise eine Linsen- und/oder Spiegelanordnung zur verbesserten Einkopplung der erzeugten Strahlung vorgesehen sein, oder es können auch fasergekoppelte Superlumineszenz- oder Laserdioden verwendet werden. Erfindungsgemäß kann die Einkopplung mit einer numerischen Apertur bis über 0,25 erfolgen, was die Verwendung einer Vielzahl gängiger Superlumineszenz- oder Laserdioden ermöglicht. Sind die Lichterzeugungsmittel in einem eigenen Gehäuse bzw. als separate Lichtquelle angeordnet, kann insbesondere ein Lichtleitkabel zur Weiterleitung der Strahlung vorgesehen sein, das mit Verbindungsmitteln zur Verbindung mit der Lichtquelle oder weiteren Lichtleitern versehen sein kann.

Ferner ist wenigstens eine endoskopisch einsetzbare Beleuchtungsoptik vorgesehen. Eine solche Beleuchtungsoptik kann flexibel, halbstarr oder starr sein und aus einer Lichtleitfaser, einem Bündel von Lichtleitfasern, einem Lichtleitstab oder einer anderen Einrichtung zur Lichtweiterleitung bestehen, die in einen Hohlraum eines Gegenstands oder eines menschlichen oder tierischen Körpers hineinbringbar ist. Die Lichtleitfasern können insbesondere Multimode-Fasern sein. Es können auch Mittel zur Verteilung der Strahlung auf den zu beobachtenden Bereich vorgesehen sein, wie beispielsweise ein Diffusor oder eine Aufweitungsoptik zur gleichmäßigen Ausleuchtung des Objektfelds, insbesondere am distalen (objektnahen) Ende der Beleuchtungsoptik. Insbesondere dann, wenn die Strahlung mit einer hohen numerischen Apertur eingekoppelt bzw. weitergeleitet wird, kann die Aufweitungsoptik ganz oder teilweise entfallen.

Zur Vermeidung der Einkopplung unerwünschter Strahlung, beispielsweise zur Verringerung der Wärmebelastung bei einer endoskopischen Anwendung in einem lebenden Körper, können weiterhin Filtermittel vorgesehen sein, die bestimmte Anteile der erzeugten Strahlung ganz oder teilweise wegfiltern. Solche Filtermittel können insbesondere auch vorteilhaft sein, um zu verhindern, dass unerwünschte Strahlung von einem Bildsensor aufgenommen wird und dadurch dessen Funktion oder die Aussagekraft des von diesem erzeugten Bilds beeinträchtigt.

Die erfindungsgemäße Vorrichtung umfasst weiterhin Lichtabbildungsmittel zur Abbildung einer Signalstrahlung aus dem zu beobachtenden Bereich mindestens auf einen phasenempfindlichen Bildsensor, wobei die Lichtabbildungsmittel mindestens eine endoskopisch einsetzbare, flexible, halbstarre oder starre Abbildungsoptik umfassen. Hierfür kann insbesondere eine Linsenanordnung, beispielsweise ein Endoskopobjektiv, vorgesehen sein. Dieses kann eine Abbildung auf einen nahe dem distalen Ende der Abbildungsoptik angeordneten Bildsensor erzeugen. Es können aber auch Bildweiterleitungsmittel vorgesehen sein, beispielsweise ein Stablinsensystem oder ein Bildleiter aus Lichtleitfasern, um das von dem Endoskopobjektiv erzeugte Bild an das proximale (objektferne) Ende der Abbildungsoptik weiterzuleiten, wo die Abbildung auf einen Bildsensor vermittelt wird. Die Lichtabbildungsmittel können weiterhin Filtermittel umfassen, um bestimmte Anteile der aufgenommenen Strahlung abzublocken.

Die Beleuchtungsoptik und die Abbildungsoptik sind in einem endoskopisch einsetzbaren Schaft (29) angeordnet, der starr, halbstarr oder flexibel ausgebildet sein kann. Der Schaft kann in bekannter Weise durch eine natürliche oder künstliche Körperöffnung in einen körperinneren Hohlraum oder auch beispielsweise durch eine Inspektionsöffnung in das Innere eines technischen Bauteils eingeführt werden. Dabei kann es aus Gründen der Handhabung vorteilhaft sein, wenn die Lichterzeugungsmittel als kompakte Einheit mit dem Schaft verbunden sind. Hierfür sind insbesondere Lichterzeugungsmittel mit geringer Verlustleistung und Baugröße geeignet, beispielsweise Superlumineszenz- oder Laserdioden.

Ferner umfasst die erfindungsgemäße Vorrichtung Steuerungsmittel zur Steuerung der Lichterzeugungsmittel und zur Ansteuerung des phasenempfindlichen Bildsensors, der als ein phasenempfindlich ansteuerbarer Festkörpersensor ausgebildet ist. Insbesondere ermöglichen die Steuerungsmittel die Erzeugung der Modulation der Messstrahlung und eine entsprechende Ansteuerung des phasenempfindlichen Bildsensors zur phasenselektiven Aufnahme der empfangenen Strahlung und zum Auslesen des Signals des phasenempfindlichen Bildsensors.

Schließlich sind erfindungsgemäß Auswertungsmittel vorgesehen zur Auswertung der von dem phasenempfindlichen Bildsensor, der eine Mehrzahl von Pixeln aufweist, gelieferten Daten, die pixelweise Phaseninformationen in Bezug auf die Modulation der Signalstrahlung enthalten, zur Erzeugung von 3D-Daten. Dabei kann aus den Phaseninformationen auf die Zeitverzögerung zwischen Auftreffen der Signalstrahlung und Aussendung der Messstrahlung geschlossen werden, um ein Bild zu erzeugen, das beispielsweise pixelweise diese Zeitverzögerung und damit die Laufzeit zum beobachteten Objekt und zurück darstellt. Ein solches Bild kann schließlich beispielsweise auf einem Videoschirm oder auch mit Hilfe von 3D-Darstellungsmitteln dargestellt werden.

Erfindungsgemäß ist mindestens ein weiterer Bildsensor vorgesehen, wobei die Lichtabbildungsmittel einen Strahlteiler und/oder einen optischen Umschalter umfassen zur Abbildung der vom Objekt zurückkommenden Strahlung auf die Bildsensoren. Die weiteren Bildsensoren können beispielsweise als CCD- oder als andere Halbleiter-Bildsensoren ausgebildet sein, etwa als UV-, IR-, Restlicht- oder weitere phasenempfindliche Bildsensoren.

Die Verwendung weiterer Sensoren ermöglicht die Gewinnung zusätzlicher Informationen aus dem beobachteten Bereich, wie etwa spektraler oder Intensitätsinformationen, oder auch eines Fluoreszenzbilds. Dadurch ist beispielsweise bei einer diagnostischen Anwendung die Erkennung von Strukturen möglich, die allein im visuellen Bild nicht oder nicht deutlich genug hervortreten.

Erfindungsgemäß ist ein Strahlteiler vorgesehen. Dieser dient zur Aufteilung der empfangenen Strahlung auf verschiedene Beobachtungsstrahlengänge bzw. Sensoren. Als Strahlteiler können insbesondere ein oder mehrere halbdurchlässige Spiegel, bevorzugt in Form teilverspiegelter Flächen in Bildteilerprismen oder Bildteilerwürfeln, verwendet werden, wobei die halbdurchlässigen Spiegel auch als dichroitische Spiegel zur spektral selektiven Bildaufteilung ausgebildet sein können. Die Verwendung halbdurchlässiger Spiegel hat den Vorteil, dass zu jedem Zeitpunkt eine Informationsgewinnung über verschiedene Beobachtungsstrahlengänge möglich ist.

Alternativ oder ergänzend hierzu können auch Mittel zum Umschalten zwischen verschiedenen Beobachtungsstrahlengängen bzw. Sensoren vorgesehen sein. Dies können mechanische Mittel sein, beispielsweise ein Chopper, oder auch elektrische Mittel, wie ein elektrisch ansteuerbarer Spiegel. Die Umschaltmittel können auch mit den Lichterzeugungsmitteln und/oder mit dem phasenempfindlichen Bildsensor synchron ansteuerbar sein. Das Umschalten ermöglicht in vorteilhafter Weise die abwechselnde Nutzung der verschiedenen Beobachtungsstrahlengänge.

Ferner ist eine Anpassungsoptik vorgesehen zur Anpassung der Gesichtsfelder des phasenempfindlichen und mindestens eines weiteren Bildsensors. Wenn aus prinzipiellen Gründen oder auch aus Platz- oder Kostengründen Bildsensoren verwendet werden, die unterschiedliche Formate aufweisen, hat dies den Vorteil, das ggf. unterschiedliche Sensorgrößen bzw. Bilddiagonalen und/oder Sensorformate nicht zu unterschiedlich großen Gesichtsfeldern führen, sondern die verschiedenen Bilddaten jeweils das gleiche Gebiet repräsentieren. Dies ist insbesondere vorteilhaft, wenn ein einfacher visueller Vergleich der verschiedenen Bilddaten durch einen Anwender ermöglicht werden soll, und dieser nicht auf das jeweils kleinste Bildfeld der unterschiedlichen Sensoren beschränkt sein soll.

Die Anpassungsoptik ist zwischen Strahlteiler und mindestens einem Bildsensor angeordnet und kann dabei aus einem optischen Glied oder mehreren bestehen und kann insbesondere Linsen mit negativer und/oder positiver Brechkraft enthalten oder entsprechende nichtplane Spiegelelemente einschließlich des halbdurchlässigen Spiegels des Strahlteilers. Hierdurch kann das auf einem Bildsensor erzeugte Bild vergrößert oder verkleinert werden, so dass es etwa das gleiche Gesichtsfeld abbildet wie auf einem oder mehreren anderen Bildsensoren. Zur Anpassung an unterschiedliche Formate bzw. Seitenverhältnisse der Bildsensoren kann die Anpassungsoptik auch astigmatische optische Elemente enthalten. Die Anpassungsoptik kann auch zusammen mit einem Bildteilerprisma oder -würfel als kompakter Block ausgebildet sein. Eine Vergrößerungs-Anpassungsoptik kann auch eine Verkleinerung des Strahlteilers und damit eine Gewichts-, Volumen- und Kostenreduktion ermöglichen.

Zur Einstellung, Fokussierung und/oder Justage kann die Anpassungsoptik auch veränderlich, beispielsweise als Zoomoptik, ausgebildet sein. Dies ist insbesondere dann vorteilhaft, wenn der phasenempfindliche und/oder der mindestens eine weitere Bildsensor vom Strahlteiler bzw. einer optischen Einheit, die den Strahlteiler umfasst, trennbar ausgebildet sind. In diesem Fall kann beim Ansetzen eines Bildsensors eine neue Einstellung der Bildebene und/oder Vergrößerung erforderlich sein. Insbesondere dann, wenn ein Bildsensor durch einen andersartigen ersetzt wird, kann durch eine Anpassungsoptik die Anpassung der jeweiligen Gesichtsfelder ermöglicht werden.

Hierdurch wird die erfindungsgemäße Aufgabe vollständig gelöst.

Bei einer bevorzugten Ausführungsform ist mindestens der phasenempfindliche Bildsensor in einer Kameraanordnung aufgenommen, die mit dem endoskopisch einsetzbaren Schaft lösbar verbunden ist. Insbesondere kann die Kameraanordnung als kompakte Kameraeinheit mit einem Gehäuse ausgebildet sein, das beispielsweise über eine Endoskop-Kupplung einfach und sicher mit dem Endoskopschaft verbindbar ist.

Gemäß einer weiteren bevorzugten Ausführungsform können die Beleuchtungsoptik, die Abbildungsoptik und der gemeinsame endoskopisch einsetzbare Schaft Teile eines Standard-Endoskops sein, das ein starres, halbstarres oder flexibles Endoskop sein kann. Ein solches Standard-Endoskop kann ein Okular für einen direkten Einblick aufweisen oder auch für den Anschluss einer Kameraeinheit mittels eines C-Mount vorbereitet sein.

Dies hat den Vorteil, dass vorhandene Endoskope erfindungsgemäß für die 3D-Datenaufnahme verwendet werden können. Solche Endoskope stehen in einer Vielzahl von Ausführungen für verschiedene human- und tiermedizinische oder technische Anwendungen zur Verfügung. Es können aber auch im distalen Bereich weitere, fest oder lösbar damit verbundene Komponenten vorgesehen sein, wie beispielsweise Strahlteiler, Bildsensor usw., die für die 3D-Datenaufnahme spezifisch sind. In diesem Fall ermöglicht die Verwendung der übrigen Komponenten eines Standard-Endoskops zumindest eine kostengünstige Herstellung, insbesondere durch Verwendung von Materialien, deren Biokompatibilität, Sterilisierbarkeit, Hitzebeständigkeit usw. erwiesen sind.

In einer weiteren bevorzugten Ausgestaltung der Erfindung sind zumindest die Abbildungsoptik, der phasenempfindliche Bildsensor, mindestens ein weiterer Bildsensor und mindestens eine Anpassungsoptik innerhalb des endoskopisch einsetzbaren Schafts nahe dessen distalem Ende angeordnet. Ferner kann auch die Beleuchtungsoptik nahe dem distalen Ende, sowie weitere optische und elektronische Bauelemente innerhalb des Schafts aufgenommen sein. Dies hat den Vorteil, dass lediglich elektrische und/oder Lichtleitkabel die Verbindung zwischen distalem und proximalem Ende des Schafts herstellen müssen, was eine besonders einfache, kostengünstige und schlanke Ausführung des Schafts ermöglicht, insbesondere eines flexiblen Schafts. In einer bevorzugten Weiterbildung der Erfindung sind zumindest die genannten Bauelemente, vorteilhafterweise auch weitere optische und elektronische Bauelemente, zu einer in Art eines Wechselobjektivs vom verbleibenden Teil des Schafts und des Endoskops trennbaren Einheit zusammengefasst. Dies hat den weiteren Vorteil, dass eine Nutzung des Endoskops auch für andere Anwendungsfälle als die Fluoreszenz-Bildgebung möglich ist.

Für die Erzeugung der Messstrahlung können Leuchtmittel unterschiedlicher Kohärenz verwendet werden. Bei Beleuchtung mit Strahlung hoher Kohärenz kann das entstehende Bild durch Speckles beeinträchtigt sein. Diese erzeugen scheinbare Strukturen in der Beleuchtungsstärke und/oder im beobachteten Signal, die die Strukturen des beobachteten Objekts überdecken können. Gemäß einer bevorzugten Ausführungsform der Erfindung sind deshalb Mittel zur Verringerung der Kohärenz vorgesehen, um ein gleichmäßiges Bild zu erzeugen. So können beispielsweise die Leuchtmittel derart angesteuert werden, dass der Kohärenzgrad unterhalb einer vorgegebenen Schwelle bleibt oder dann, wenn im erzeugten Bild in erheblichem Maße Speckles auftreten, der Kohärenzgrad reduziert wird. Dies kann beispielsweise bei einer Laserdiode durch Betrieb an bzw. dicht unter der Laserschwelle erreicht werden, es kann aber auch die Laserdiode derart angesteuert bzw. moduliert werden, dass die Kohärenz der emittierten Strahlung geringer ist.

Bei einer erfindungsgemäßen Vorrichtung zur 3D-Datenaufnahme kann auch ein Okular vorgesehen sein. Der direkte Einblick durch ein Okular hat den Vorteil, dass auch schon unabhängig von der Bilderzeugung durch Bildsensoren eine Beobachtung des zu beobachtenden Gebiets möglich ist, um beispielsweise einem Benutzer in der gewohnten Weise eine einfache Orientierung in einem Körperhohlraum bzw. in einem technischen Bauteil zu ermöglichen.

Gemäß einer weiteren bevorzugten Ausführungsform der Erfindung ist ein Bildsensor vorgesehen, der zur Erzeugung eines Bilds des beobachteten Gebiets mittels des reflektierten bzw. gestreuten Lichts ausgebildet ist. Ein solches Bild hat den Vorteil, dass es beispielsweise bei einer diagnostischen Anwendung den gewohnten Anblick des zu diagnostizierenden Gewebebereichs wiedergibt und dadurch die Orientierung erleichtert. Zur Unterscheidung zwischen reflektiertem und gestreutem Licht können auch polarisationsoptische Elemente vorgesehen sein.

Gemäß einer weiteren bevorzugten Ausführungsform der Erfindung kann ein spektral aufgelöstes bzw. selektiertes Bild des beobachteten Bereichs erzeugt werden. Hierfür können beispielsweise Filter vorgesehen sein, die nur einen bestimmten spektralen Anteil des reflektierten bzw. gestreuten Lichts hindurch lassen. Ein spektral in mehrere Anteile aufgelöstes bzw. nach einem spektralen Anteil selektiertes Bild erlaubt eine kontrastreichere Darstellung und verbessert dadurch die Sicherheit einer Diagnose.

Insbesondere für den Fall einer schmalbandigen spektral selektiven Bildgebung kann ein spektral selektives Element, wie ein Prisma oder ein Gitter vorgesehen sein. Ein Abbild des beobachteten Bereichs entsteht dann durch Punkt- oder Linienscannen. Hierdurch kann ein besonders kontrastreiches Bild erzeugt werden.

Gemäß einer weiteren bevorzugten Ausführungsform ist ein Bildsensor zur Erzeugung eines Fluoreszenzbilds vorgesehen. Der weitere Bildsensor kann insbesondere in an sich bekannter Weise als Bildsensor für ein PDD-System ausgebildet sein. Das erzeugte PDD-Bild kann dabei auch einen Anteil des reflektierten bzw. gestreuten Lichts enthalten. Das Fluoreszenzbild kann auch ein Fluoreszenzlebensdauerbild sein, das auch mit Hilfe der 3D-Daten in Bezug auf die Entfernung der fluoreszierenden Oberfläche korrigiert sein kann. Das Fluoreszenzbild steht als ergänzende Darstellung des beobachteten Gebiets zur Verfügung. Hierdurch ist beispielsweise eine Vermessung krankhafter Veränderungen in einem biologischen Gewebe mit größerer Sicherheit möglich.

Gemäß einer weiteren bevorzugten Ausführungsform sind die 3D-Daten zur Längen-, Flächen- und/oder Volumenmessung auswertbar. Aus einem 2D-Bild des beobachteten Bereichs kann unter Zuhilfenahme der Entfernungsinformationen die laterale Ausdehnung einer Struktur ermittelt werden, sowie aus den Entfernungsdaten die Tiefenausdehnung der Struktur. Auf diese Weise ist die Messung von Flächen und z. T. auch Volumina möglich. Dies ist insbesondere bei endoskopischen Anwendungen vorteilhaft, wo eine Tiefeninformation, die für die Ermittlung des Abbildungsmaßstabs notwendig ist, nicht ohne weiteres vorhanden ist. Sowohl bei der Inspektion technischer Bauteile als auch bei der medizinischen Diagnose von Gewebeveränderungen sind solche Messungen wünschenswert zur Bestimmung der Größe und, bei mehreren Messungen, zeitlichen Veränderung von Strukturen wie Rissen in technischen Bauteilen oder Läsionen oder Tumoren in menschlichen oder tierischen Organen. In besonders vorteilhafter Weise kann die Messung von Flächen oder Volumina kombiniert werden mit einem Fluoreszenzbild.

In einer weiteren bevorzugten Ausführungsform der Erfindung wird aus den 3D-Daten ein Stereobild synthetisiert. Ein solches Stereobild ist mit einer geeigneten Anzeigeeinrichtung, die einem Betrachter für beide Augen jeweils unterschiedliche Bilder anzeigt, darstellbar und vermittelt dem Betrachter einen wirklichkeitsnahen räumlichen Eindruck vom beobachteten Gebiet. Dies ist insbesondere wünschenswert bei endoskopischen Operationen zur Verbesserung der Haptik, d. h. der intuitiven Kontrolle des Operateurs über die von ihm bedienten Instrumente. Da die synthetische Stereobasis nahezu beliebig wählbar ist und lediglich durch die Vermeidung von Hinterschneidungen begrenzt wird, kann hierbei der Stereoeffekt auf beliebige endoskopische Operationssituationen eingestellt werden, auch beispielsweise für robotergestützte Operationen. Dabei können die mechanischen Übersetzungsverhältnisse der Manipulatoren, der optische Abbildungsmaßstab und der Stereoeffekt so eingestellt werden, dass sie zueinander passen.

Für die Berechnung des linken bzw. rechten Stereobilds werden, ausgehend von einem 2D-Bild, beispielsweise einem Farb- oder Grauwertbild, im linken Bild alle Pixel individuell nach rechts versetzt mit einem Versatz, der ungefähr umgekehrt proportional zum Abstand ist, der mit dem 3D-Sensor ermittelt wurde. Entsprechend werden im rechten Bild alle Pixel nach links verschoben. Bei Bedarf können noch eine Pixelglättung und eine Korrektur der Verzeichnung erfolgen. Diese Bildverarbeitungsoperationen lassen sich auch am laufenden Videobild in Echtzeit durchführen. Die beiden Bilder können dann beispielsweise auf einem Stereo-Bildschirm oder mit einem Stereo-Betrachtungsgerät dargestellt werden.

Die erfindungsgemäße Vorrichtung kann gemäß einer besonders bevorzugten Ausführungsform derart ausgebildet sein, dass die von dem phasenempfindlichen und mindestens einem weiteren Bildsensor gelieferten Bilddaten oder auch von einer am Okular angeschlossenen Kamera gelieferten Bilddaten in einer synoptischen Weise dargestellt werden können. Eine solche Darstellung kann eine überlagerte Darstellung der betreffenden Bilddaten sein, eine abwechselnde bzw. eine Darstellung nacheinander oder eine Darstellung in nebeneinander angeordneten Bildern. Für die Darstellung für einen Benutzer können entsprechende Anzeigeeinrichtungen vorgesehen sein, wie beispielsweise ein oder mehrere Videoschirme, Projektoren, Drucker usw.. Ebenso können Speichermittel zur Aufzeichnung, Dokumentation und Archivierung der Bilddaten vorgesehen sein. Die Steuerungs- und Auswertemittel können ferner zur Durchführung von Bildanalyseverfahren, die vom Benutzer unabhängig oder von diesem einstellbar sein können, ausgebildet sein. Hierdurch können beispielsweise Kontraste hervorgehoben oder verringert werden, die Daten geglättet oder auch Strukturen aus den von verschiedenen Bildsensoren gelieferten Bilddaten miteinander korreliert werden. So können beispielsweise Informationen aus einem visuellen bzw. RGB-Bild, aus AF- bzw. PDD-Bildern und/oder einem Fluoreszenzbild mit den 3D-Daten und/oder miteinander kombiniert werden.

Die Steuerungs- und Auswerteeinrichtung kann auch dazu ausgebildet sein, durch Korrelation der Bilddaten der verschiedenen Bildsensoren automatisch eine lagerichtige Überlagerung der Bilddaten herzustellen oder auch eine automatische Anpassung der Gesichtsfelder bezüglich Lage, Größe und/oder Format durchzuführen. Ebenso kann, ggf. sensorabhängig, eine automatische Fokussierung oder eine automatische Verzeichnungskorrektur vorgesehen sein.

Gemäß einer weiteren bevorzugten Ausführungsform der Erfindung kann es vorgesehen sein, nicht nur solches Licht zu erzeugen und zu dem zu beobachtenden Gebiet weiterzuleiten, das als Messstrahlung geeignet ist, sondern noch mindestens eine weitere Strahlung. Diese kann beispielsweise weißes Licht sein zur Erzeugung eines Weißlichtbildes bei Beobachtung durch ein Okular oder mittels eines entsprechenden weiteren Bildsensors. Hierfür können entsprechende Lichterzeugungsmittel vorhanden sein, beispielsweise eine Xenon- oder Metall-Halid-Lampe zur Erzeugung einer Weißlichtbeleuchtung, die in einer eigenen Lichtquelle oder zusammen mit den Lichterzeugungsmitteln für die Messstrahlung in einer gemeinsamen Lichtquelle angeordnet sein können.

Ebenso können für die Erzeugung einer breitbandigen Beleuchtung auch fasergepumpte Fluoreszenzlichtquellen, wie beispielsweise in US 20070092184 A1 offenbart, verwendet werden, wenn diese genügend kurze Fluoreszenzlebensdauern aufweisen. Bei der Verwendung von Superkontinuum-Weißlichtlaserquellen ergibt sich der zusätzliche Vorteil, dass diese, ggf. synchron mit dem phasenempfindlichen Bildsensor, modulierbar sind. Darüber hinaus können Superkontinuum-Weißlichtlaser wellenlängenselektiv strahlen, und sind entsprechend elektrisch ansteuerbar. Eine solche Lichtquelle kann zur Erzeugung aller benötigten Strahlungsarten ausreichen.

Häufig kann für die Erzeugung eines Bilds im reflektierten bzw. gestreuten Licht die Messstrahlung selbst verwendet werden. In vorteilhafter Weise kann für die Erzeugung eines solchen Bildes aber auch eine breitbandige, insbesondere eine Weißlichtbeleuchtung, beispielsweise durch eine Xenon-Lichtquelle, verwendet werden.

Die weitere Strahlung kann gleichzeitig mit der Messstrahlung erzeugt und in das zu beobachtende Gebiet weitergeleitet werden oder auch durch entsprechende Ansteuerung und/oder durch ein optisches Schaltelement wechselweise mit der Messstrahlung erzeugt bzw. weitergeleitet werden. Letzteres ist insbesondere dann vorteilhaft, wenn nicht zu allen Zeiten eine Beleuchtung mit den jeweiligen Strahlungen notwendig ist, und in den anderen Zeiten eine Abschaltung bzw. Abblockung der jeweils nicht benötigten Strahlung erfolgen kann, um die eingekoppelte Energiemenge zu reduzieren. Die Umschaltfrequenz kann so hoch gewählt werden, dass die Umschaltung mit dem Auge oder auf der Anzeigeeinrichtung nicht mehr oder nur noch wenig bemerkbar ist. Insbesondere kann die Wechselfrequenz so mit der Bildfrequenz synchronisiert werden, dass eine ganze Anzahl Videobilder auf eine halbe Wechselperiode fällt.

Ein erfindungsgemäßes Verfahren zur endoskopischen 3D-Datenerfassung umfasst die folgenden Schritte:
- Erzeugung mindestens einer modulierten Messstrahlung,
- Weiterleitung der Messstrahlung auf einen zu beobachtenden Bereich durch mindestens eine endoskopisch einsetzbare Beleuchtungsoptik,
- Abbildung einer Signalstrahlung aus dem zu beobachtenden Bereich mindestens auf einen phasenempfindlichen Bildsensor, der eine Mehrzahl von Pixeln aufweist, durch mindestens eine endoskopisch einsetzbare Abbildungsoptik,
- wobei die Beleuchtungsoptik und die Abbildungsoptik in einem endoskopisch einsetzbaren Schaft angeordnet sind, und
- Erzeugung von 3D-Daten durch Auswertung der von dem phasenempfindlichen Bildsensor gelieferten Daten,
- wobei mindestens ein weiterer Bildsensor und ein Strahlteiler und/oder ein optischer Umschalter vorgesehen sind, und
- mindestens eine Anpassungsoptik vorgesehen ist zur Anpassung der Gesichtsfelder der Bildsensoren.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1 ein bevorzugtes Ausführungsbeispiel einer erfindungsgemäßen Vorrichtung;
Fig. 2 eine alternative Ausführungsform eines in einer erfindungsgemäßen Vorrichtung verwendeten Strahlteilers;
Fig. 3 eine erfindungsgemäße Lichtquelle;
Fig. 4 eine weitere Ausführungsform einer erfindungsgemäßen Vorrichtung;
Fig. 5 ein vergrößertes Detail aus Fig. 4.

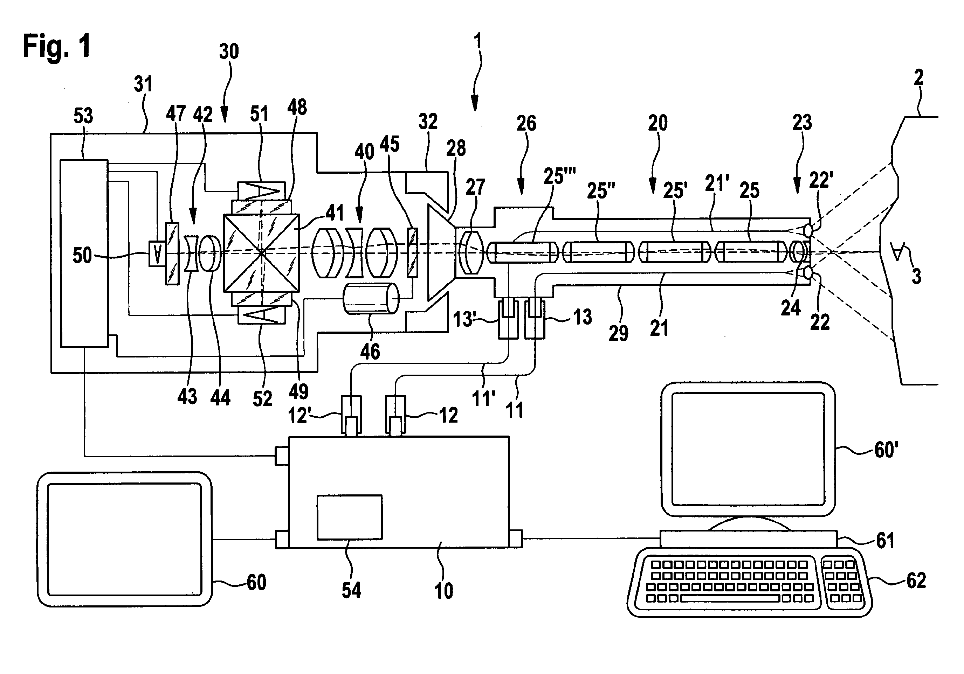

Gemäß Fig. 1 umfasst eine erfindungsgemäße Vorrichtung 1 eine Lichtquelle 10. In dem dargestellten bevorzugten Ausführungsbeispiel ist die Lichtquelle zur Erzeugung einer kontinuierlich, beispielsweise sinusförmig, modulierten Messstrahlung und einer Weißlichtbeleuchtung ausgebildet. Zur Weiterleitung beider Strahlungsarten sind jeweils Lichtleitkabel 11, 11' vorgesehen, die über Anschlüsse 12, 12' mit der Lichtquelle 10 und über Anschlüsse 13, 13' mit dem Endoskop 20 verbunden werden können. Bei einer entsprechenden Einkopplung oder bei Verwendung eines Superkontinuum-Lasers können beide Strahlungsarten auch über einen einheitlichen Lichtleiter weitergeleitet werden. Die Lichtquelle kann auch in das Endoskop integriert sein.

Über die Endoskop-Lichtleiter 21, 21' werden die Messstrahlung und das Weißlicht zum objektseitigen (beobachterfernen, distalen) Ende 23 des Endoskops 20 geführt. Die dort angeordneten Aufweitungsoptiken 22, 22' dienen der gleichmäßigen Verteilung der Beleuchtungsstrahlung auf das zu beobachtende Gebiet, beispielsweise ein Objekt 2, etwa ein Gewebebereich in einem körperinneren Hohlraum (nicht dargestellt).

Zur Erzeugung eines Bilds eines Teilbereichs des Objekts 2 umfasst das Endoskop 20 ein Endoskopobjektiv 24. Das von diesem erzeugte Zwischenbild wird von einem Relaislinsensystem, hier einer Anordnung von Stablinsen 25, 25', 25", 25"', an das objektferne (beobachternahe, proximale) Ende 26 des Endoskops 20 geführt. Wird statt des dargestellten starren Endoskops 20 ein flexibles Endoskop verwendet, so kann das erzeugte Bild über Glasfaser-Bildleiter übertragen werden. Am proximalen Ende des Endoskops ist eine Okularlinsenanordnung 27 vorgesehen. Solange die Kameraanordnung 30 nicht an das Endoskop 20 angesetzt ist, ist ein direkter Einblick durch die Okularmuschel 28 möglich. Endoskop-Lichtleiter 21, 21', Endoskopobjektiv 24 und Relaislinsen 25, 25', 25", 25'''sind dabei in einem in eine Körperöffnung einführbaren Endoskopschaft 29 aufgenommen.

Zur 3D-Datengewinnung kann an das Endoskop 20 die Kameraanordnung 30 angesetzt werden. Diese enthält in einem Gehäuse 31 einen oder mehrere optische Sensoren, sowie weitere optische und ggf. elektronische Bauelemente. Zur lösbaren Verbindung mit dem Endoskop 20 ist eine Endoskopkupplung 32 vorgesehen, die beispielsweise einen Schnapp- oder Bajonettmechanismus enthält, um eine einfache, sichere und lösbare Verbindung zu gewährleisten. Insbesondere kann eine Anschlagfläche (nicht dargestellt) vorgesehen sein, um die relative Position von Endoskop und Kameraanordnung zu fixieren, und ein Eintrittsfenster (nicht dargestellt) zur Verhinderung der Verschmutzung der Kameraanordnung.

Die Kameraanordnung 30 enthält ein Kameraobjektiv 40, einen als Teilerwürfel 41 ausgebildeten Strahlteiler zur Aufteilung des Bilds auf mehrere Bildsensoren, Bildsensoren 50, 51, 52 und eine Anpassungsoptik 42, hier exemplarisch bei dem Bildsensor 50 dargestellt. Die Anpassungsoptik 42 enthält beispielsweise eine Linse bzw. Linsengruppe mit negativer Brechkraft 43 und eine Linse bzw. Linsengruppe mit positiver Brechkraft 44 und ist so ausgebildet, dass auf die aktive Fläche des Bildsensors 50 trotz unterschiedlicher Größe das gleiche Bildfeld abgebildet wird wie auf die Bildsensoren 51 und 52. Dies ist in Fig. 1 symbolisch durch die abgebildete Struktur 3 dargestellt, die durch die als Verkleinerungsoptik ausgebildete Anpassungsoptik auf den kleineren Bildsensor 50 kleiner als auf die Bildsensoren 51 und 52 abgebildet wird.

Ferner enthält die Kameraanordnung Aufbereitungsoptiken 45, 47, 48, 49, die insbesondere als Filter und/oder als optische Schalter ausgebildet sein können. Die Aufbereitungsoptiken können beispielsweise Farbfilter, insbesondere Absorptions- oder Interferenzfilter, elektronisch abstimmbare Filter, Prismen, Verlängerungsplatten oder Raumfrequenzfilter (Anti-Aliasing Filter) umfassen. So kann insbesondere die Aufbereitungsoptik 45, die allen Bildsensoren vorgeschaltet ist, bestimmt sein, Lichtanteile, die für alle Bildsensoren unerwünscht sind, zu eliminieren. Der Aufbereitungsoptik 45 kann ein Aktuator 46, beispielsweise zum Ein- oder Ausschwenken eines Filters, zugeordnet sein. Die Aufbereitungsoptiken 47, 48, 49 dienen der insbesondere der Filterung des auftreffenden Lichts entsprechend der jeweiligen Art des Sensors.

So kann beispielsweise der Bildsensor 50 als phasenempfindlicher Festkörper-Bildsensor ausgebildet sein, der pixelweise die Intensität der auftreffenden Signalstrahlung und die Phasenverschiebung, d. h., die Zeitverzögerung zwischen Messstrahlung und auftreffender Signalstrahlung registriert. Aus den von diesem Bildsensor gelieferten Daten können 3D-Daten rekonstruiert werden. Durch eine entsprechende Auswertung kann, wie beispielsweise in EP 1 746 410 A1 angegeben, pixelweise die Phasenverschiebung und damit die Zeitverzögerung, die der Signallaufzeit entspricht, ermittelt werden.

Der Bildsensor 51 erzeugt im Ausführungsbeispiel ein RGB-Farbbild. Die Aufbereitungsoptik 48 kann hierfür beispielsweise als IR-Sperrfilter ausgebildet sein. Der Bildsensor 52 kann beispielsweise ein Bild im NIR-Bereich erzeugen, könnte aber auch beispielsweise ein Wärmebild aufnehmen oder einen Bildverstärker zur Erhöhung der Empfindlichkeit enthalten.

Die Aufbereitungsoptiken können auch als elektronisch kontrollierbare Filter ausgebildet sein, beispielsweise als durchstimmbarer Flüssigkristallfilter (Liquid Crystal Tunable Filter, LCTF) oder als akustooptischer durchstimmbarer Filter (Acousto-Optical Tunable Filter, AOTF). Der Strahlteiler kann auch, beispielsweise durch eine dichroitische Beschichtung, spektral selektiv ausgebildet sein, so dass ein geringerer Anteil des Lichts in Sperrfiltern verloren geht.

Die Bildsensoren 50, 51, 52 sind mit einer Steuerungselektronik 53 verbunden, die die Bildsensoren kontrolliert und die Bilddaten ausliest und, ggf. nach einer ersten Vorverarbeitung, weiterleitet. Die Steuerungselektronik 53 ist auch mit der Lichtquelle 10 verbunden, so dass das Auslesen der Daten und die Modulation der Messstrahlung synchronisiert werden können. Ferner ist ein Steuergerät 54 vorgesehen, das beispielsweise in die Lichtquelle 10 integriert sein kann. Weiterhin sind Anzeigeeinrichtungen 60, 60' vorgesehen zur Anzeige der Bilddaten, sowie eine Eingabeeinrichtung, beispielsweise eine Tastatur 62, ein Touch Screen oder auch eine Spracherkennungseinrichtung.

Das Steuergerät 54 verarbeitet die Bildsignale für eine unmittelbare Anzeige, steuert die Lichtquelle 10 synchron mit den Bildsensoren, steuert ggf. ansteuerbare Filter und leitet die Bilddaten zur weiteren Verarbeitung, Anzeige und Speicherung weiter an einen Computer 61. Ferner ist im Steuergerät oder im Computer 61 eine Möglichkeit zur Verknüpfung bzw. synoptischen Darstellung der verschiedenen Bilddaten vorgesehen, sowie ggf. die Erzeugung eines synthetischen Stereobilds.

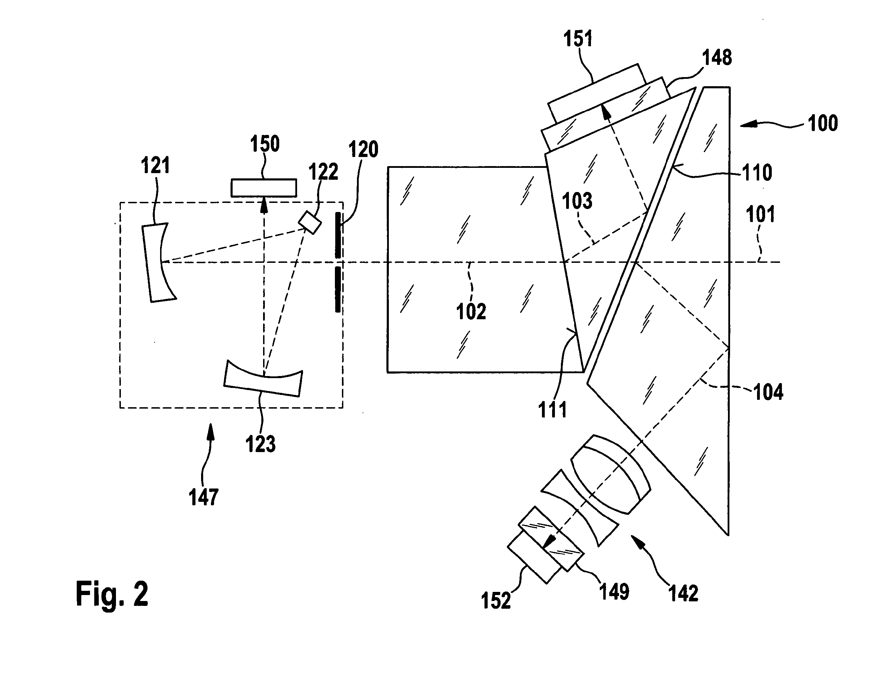

Eine alternative Ausgestaltung des Strahlteilers ist in Fig. 2 dargestellt. Anstelle des Strahlteilerwürfels 41 sind hier Prismen zur Aufteilung des Lichts vorgesehen, die einen Prismenblock 100 bilden.

Der einfallende Strahl 101 wird an zwei Grenzflächen 110 und 111 in insgesamt drei Kanäle 102, 103 und 104 aufgespalten. Diese Kanäle führen das Licht auf drei Bildsensoren 150, 151, 152, wovon mindestens einer ein phasenempfindlicher Bildsensor ist. Die einfallende Intensität kann bei metallischer Beschichtung farbneutral in vorbestimmten Intensitätsverhältnissen in die einzelnen Kanäle gespiegelt werden, bei dichroitischer Beschichtung auch farbselektiv oder spektral zerlegt. Durch Einführung zusätzlicher Flächen können auch mehr Kanäle erzeugt werden, durch ein einfaches Teilerprisma auch nur zwei Kanäle.

Vor dem Bildsensor 152 ist hier eine Anpassungsoptik 142 vorgesehen, sowie eine Aufbereitungsoptik 149, beispielsweise ein Filter. Auch die Aufbereitungsoptik vor dem Bildsensor 151 kann beispielsweise ein Filter 148 sein. Als Aufbereitungs- und Anpassungsoptik vor dem Bildsensor 150 dient in diesem Ausführungsbeispiel ein gekreuztes Czerny-Turner-Spektrometer 147. Durch die Linienblende 120 wird beispielsweise die mittlere Bildzeile selektiert und über den ersten Hohlspiegel 121, ein Beugungsgitter 122 oder auch ein diffraktives optisches Element oder ein Prisma und über den zweiten Hohlspiegel 123 auf den Bildsensor 150 abgebildet. Hierdurch kann ein spektral aufgelöstes und in einer Dimension räumlich aufgelöstes Bild erzeugt werden. Ein vollständiges Bild kann mosaikartig zusammengesetzt werden, indem die abgebildete Zeile über das Objekt geführt wird, beispielsweise durch Hin- und Herschwenken des Endoskops, was manuell oder motorisch durch eine entsprechende Schwenkeinrichtung beispielsweise bei einem Schwenkblick-Endoskop geschehen kann, oder durch automatisches Scannen durch Bewegung der Linienblende 120 und/oder des Spektrometers 147. Hierfür ist im Steuergerät eine entsprechende Steuerungs- und Auswertungssoftware installiert.

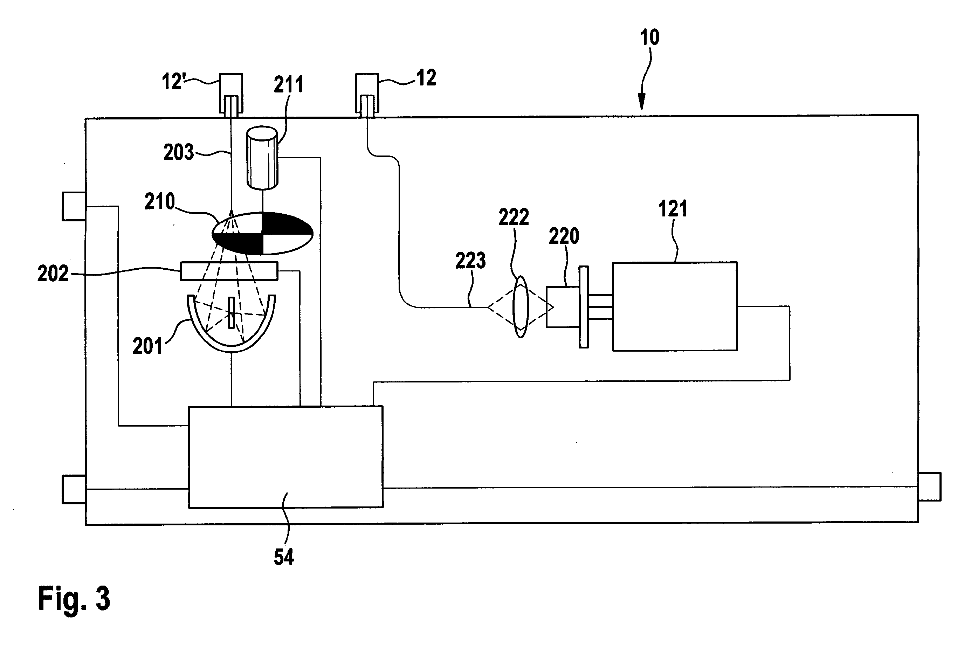

Wie in Fig. 3 dargestellt, kann erfindungsgemäß die Lichtquelle 10 das Steuergerät 54 enthalten, das mit der Steuerelektronik 53, Anzeigeeinrichtungen, wie beispielsweise einem Bildschirm 60, sowie weiteren Geräten, etwa einem Computer 61 kommuniziert und hierfür über Steckverbindungen verbindbar ist. Das Steuergerät steuert ferner die Lichterzeugung, beispielsweise von Weißlicht und der Messstrahlung.

Hierfür enthält die Lichtquelle 10 als Weißlichtquelle eine Metall-Halid-Bogen-Entladungslampe 201, die einen Reflektor umfassen kann, sowie weitere Elemente zur Kollimation bzw. Einkopplung in einen Lichtleiter 203. Alternativ können auch LED-, Xenon- oder Halogenlampen als Weißlichtquelle verwendet werden, ebenso wie RGB- oder Superkontinuum-Laserquellen. Um zu verhindern, dass das Weißlicht die Tiefenmessung beeinträchtigt, ist ein Chopperrad 210 vorgesehen, das den Lichtfluss unterbricht, sobald eine Abstandsdatenerfassung stattfindet. Dies kann manuell eingegeben werden, um abwechselnd im Weißlicht und im Messlicht zu beobachten oder auch ein Fluoreszenzbild aufzunehmen. Es kann aber auch automatisch, insbesondere im Videotakt oder einem Bruchteil davon, zwischen Weißlicht bzw. Fluoreszenzbeobachtung und 3D-Messung umgeschaltet werden. Das Steuergerät steuert den Antrieb 211 des Chopperrads entsprechend den jeweiligen Anforderungen, beispielsweise synchron mit dem Auslesen des jeweiligen Bildsensors. Anstelle eines Chopperrads kann auch ein Schwingspiegel oder ein elektronisch gesteuertes Filter verwendet werden. Bei Verwendung von Festkörperlichtquellen, beispielsweise LED- oder Laserlichtquellen können diese direkt im entsprechenden Takt angesteuert werden. Der Lichtleiter 203 leitet das Licht über einen Anschluss 12' in ein Lichtleitkabel ein.

Zur Erzeugung der Messstrahlung ist eine Laserdiode 220 vorgesehen, die über eine Treiberelektronik 221 angesteuert wird und deren Licht über eine Kollimationsoptik 222 in einen Lichtleiter 223 eingekoppelt wird. Stattdessen kann auch eine fasergekoppelte Leuchtdiode oder eine Superlumineszenzdiode eingesetzt werden. Das erzeugte Licht wird über einen Anschluss 12 in ein Lichtleitkabel zur Weiterleitung in das Endoskop eingeleitet. Die Laserdiode wird durch das Steuergerät synchron zum Auslesen des phasenempfindlichen Bildsensors moduliert.

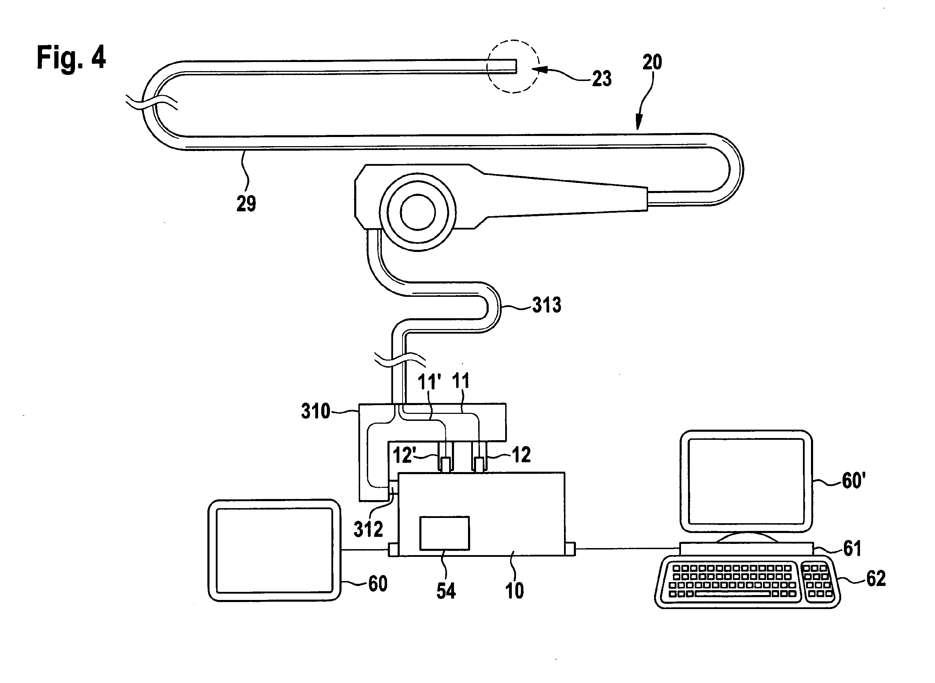

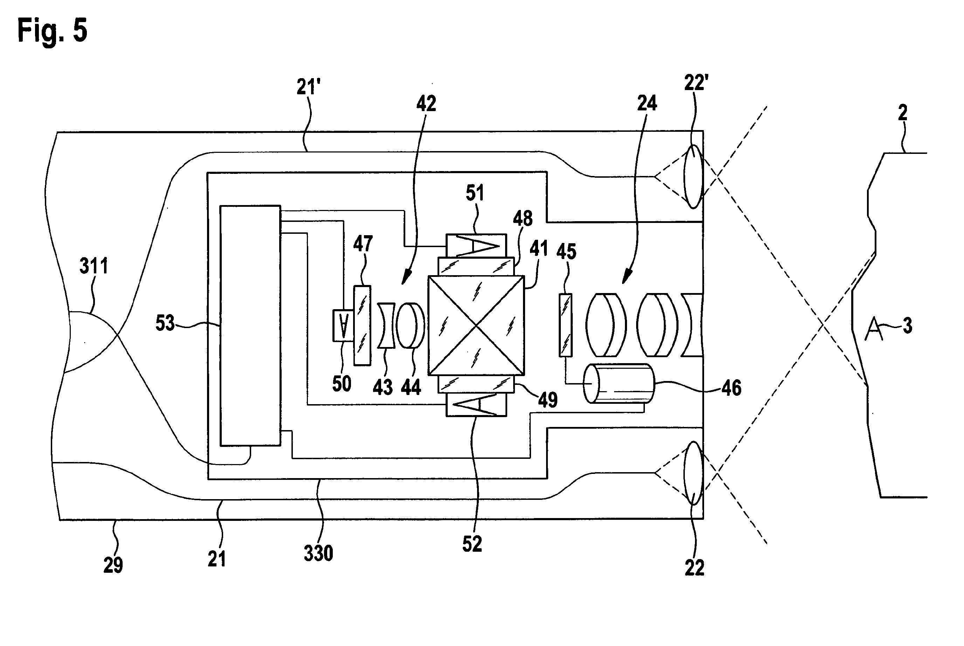

In der in Fig. 4 gezeigten weiteren Ausführungsform der Erfindung sind die optischen und elektronischen Bauelemente, die in dem in Fig. 1 dargestellten Ausführungsbeispiel der Kameraanordnung 30 zugeordnet sind, innerhalb des Endoskopschafts 29 nahe dessen distalem Ende 23 angeordnet, das in Fig. 5 im Detail dargestellt ist. Eine Relaislinsen-Optik bzw. ein Glasfaser-Bildleiter zur Weiterleitung des vom Endoskopobjektiv erzeugten Bilds an das proximale Ende des Endoskops sowie ein Okular sind hier nicht notwendig. Das Endoskopobjektiv 24 umfasst hier die Gesamtheit der Linsen vor dem Strahlteiler 41, ein zusätzliches Kameraobjektiv ist nicht notwendig. Die Aufbereitungsoptik 45, beispielsweise ein einschwenkbarer Filter, ist hier beispielsweise zwischen Endoskopobjektiv 24 und Strahlteiler 41 angeordnet. Das Endoskopobjektiv 24 und die der Kameraanordnung 30 zuzuordnenden Bauelemente können zu einer distalen Kameraeinheit 330 zusammengefasst sein, die über eine Kupplung (nicht dargestellt) lösbar mit dem verbleibenden Teil des Endoskops 20 verbunden sein kann und in der Art eines Wechselkopfs bzw. -objektivs gegen anderen Kameraeinheiten austauschbar sein kann. Die distale Kameraeinheit 330 kann auch die distale Beleuchtungsoptik, insbesondere die Aufweitungsoptiken 22, 22' und diesen zugeordnete Teile der Endoskop-Lichtleiter 21, 21', sowie das distale Ende des Endoskopschafts 29 umfassen.

Der Endoskopschaft 29 ist in diesem Ausführungsbeispiel flexibel, kann aber auch halbstarr oder starr sein. Die Steuerungselektronik 53 ist über ein Kabel 311, das gemeinsam mit den Lichtleitkabeln 11, 11' durch den Endoskopschaft 29 und ein Endoskopkabel 314 geführt ist, mit dem Steuergerät 54 verbunden. Zur Verbindung des Endoskops 20 mit der Lichtquelle 10 und mit dem Steuergerät 54 können die Anschlüsse 12, 12' und der Anschluss 312 des Kabels 54 in eine Anschlussbox 310 zusammengefasst sein.

Vorteilhafterweise entspricht der Aufbau des Endoskops 20 dem eines Standard-Videoskops. Zur Beschreibung weiterer Einzelheiten wird auf Fig. 1 verwiesen.

Soll ein erfindungsgemäßes Verfahren zur endoskopischen 3D-Datenerfassung angewendet werden, so wird das Endoskop 20 in die Körperhöhle oder in das technische Bauteil, worin die Messung erfolgen soll, eingeführt. Die Kameraanordnung 30 wird, sofern sie nicht eine Einheit mit dem Endoskop darstellt oder bereits mit diesem verbunden ist, an das Endoskop 20 angeschlossen, beispielsweise über die Endoskopkupplung 32 an die Okularmuschel 28 angeclippt. Sofern die Lichterzeugungsmittel nicht Teil des Endoskops sind, wird die Lichtquelle 10 mit dem Endoskop 20 über die Lichtkabel 11, 11' verbunden. Vorteilhafterweise wird das Endoskop 20 an einem Halter befestigt, der eine Bewegung des Endoskops relativ zum untersuchten Objekt während der Untersuchung verhindert.

Zur Durchführung des erfindungsgemäßen Verfahrens zur 3D-Datenerfassung wird in der Lichtquelle 10 das Beleuchtungslicht erzeugt, insbesondere die Messstrahlung und Weißlicht. Das Weißlicht kann beispielsweise das ganze sichtbare Spektrum oder einen Teil davon umfassen, kann aber auch aus einem oder mehreren schmalbandigen Anteilen bestehen. Die Messstrahlung ist mit einer Frequenz von beispielsweise ca. 10 - 100 MHz sinusförmig intensitätsmoduliert. Vorteilhafterweise ist das Weißlicht im Videotakt schaltbar.

Weißlicht und Messstrahlung werden über die Lichtkabel 11, 11' und die Lichtleiter 21, 21' auf das zu beobachtende Gebiet geleitet. Über die Abbildungsoptik wird auf dem phasenempfindlichen Bildsensor 50 ein Bild erzeugt. Durch mit der Modulation der Messstrahlung synchronisiertes Auslesen des Bildsensors werden pixelweise phasenabhängige Daten gewonnen, die von der Steuerelektronik 53 und dem Steuergerät 54 zu Intensitäts- und Phaseninformationen verarbeitet werden. Das Steuergerät erzeugt dadurch Tiefeninformationen, die die Zeitverzögerung der Signalstrahlung gegenüber der Messstrahlung darstellen, und daraus wiederum 3D-Daten. Ebenso kann ein Fluoreszenzbild erzeugt werden.

Die 3D-Daten können für den Benutzer auf geeigneten Anzeigeeinrichtungen dargestellt werden und für weitere Bildverarbeitungsschritte oder für die Speicherung zur Verfügung stehen. Auch beispielsweise ein RGB- oder ein Fluoreszenzbild können zu diesem Zweck beispielsweise abwechselnd oder in Überlagerung mit den 3D-Daten dargestellt werden.

## Patentansprüche

1. Vorrichtung zur endoskopischen 3D-Datenerfassung mit:
- Lichterzeugungsmitteln zur Erzeugung mindestens einer modulierten Messstrahlung,
- Lichtweiterleitungsmitteln zur Weiterleitung der Messstrahlung auf einen zu beobachtenden Bereich, wobei die Lichtweiterleitungsmittel eine endoskopisch einsetzbare Beleuchtungsoptik umfassen,
- Lichtabbildungsmitteln zur Abbildung einer Signalstrahlung aus dem zu beobachtenden Bereich mindestens auf einen phasenempfindlichen Bildsensor (50), der eine Mehrzahl von Pixeln aufweist, wobei die Lichtabbildungsmittel eine endoskopisch einsetzbare Abbildungsoptik umfassen,
- wobei die Beleuchtungsoptik und die Abbildungsoptik in einem endoskopisch einsetzbaren Schaft (29) angeordnet sind, und
- Steuerungs- und Auswertungsmitteln zur Steuerung der Lichterzeugungsmittel, zur Ansteuerung des phasenempfindlichen Bildsensors und zur Auswertung der von dem phasenempfindlichen Bildsensor gelieferten Daten zur Erzeugung von 3D-Daten,
- wobei mindestens ein weiterer Bildsensor (51, 52) vorgesehen ist und die Lichtabbildungsmittel einen Strahlteiler (41) zur Aufteilung der empfangenen Strahlung auf verschiedene Sensoren und/oder einen optischen Umschalter zum Umschalten zwischen verschiedenen Sensoren umfassen,
**dadurch gekennzeichnet, dass**
- die Bildsensoren (50, 51, 52) unterschiedliche Sensorgrößen und/oder Sensorformate aufweisen und dass
- mindestens eine Anpassungsoptik (42) vorgesehen ist zur Anpassung der Gesichtsfelder der Bildsensoren (50, 51, 52), so dass das auf einem Bildsensor (50, 51, 52) erzeugte Bild das gleiche Gesichtsfeld abbildet wie auf einem oder mehreren anderen Bildsensoren (50, 51, 52).

2. Vorrichtung nach Anspruch 1, **dadurch gekennzeichnet, dass** eine Kameraanordnung (30) vorgesehen ist, die mindestens den phasenempfindlichen Bildsensor (50) enthält, und die mit dem endoskopisch einsetzbaren Schaft (29) lösbar verbunden ist.

3. Vorrichtung nach Anspruch 2, **dadurch gekennzeichnet, dass** die Beleuchtungsoptik, die Abbildungsoptik und der gemeinsame endoskopisch einsetzbare Schaft (29) Teile eines Standard-Endoskops (20) sind.

4. Vorrichtung nach einem der Ansprüche 1 bis 3, **dadurch gekennzeichnet, dass** zumindest die Abbildungsoptik, der phasenempfindliche Bildsensor (50), mindestens ein weiterer Bildsensor (51, 52) und mindestens eine Anpassungsoptik (42) innerhalb des endoskopisch einsetzbaren Schafts (29) nahe dessen distalem Ende angeordnet sind.

5. Vorrichtung nach einem der Ansprüche 1 bis 4, **dadurch gekennzeichnet, dass** Mittel zur Kohärenzreduktion vorgesehen sind.

6. Vorrichtung nach einem der Ansprüche 1 bis 5, **dadurch gekennzeichnet, dass** ein Bildsensor zur Erzeugung eines Bilds mittels des reflektierten und/oder gestreuten Lichts vorgesehen ist.

7. Vorrichtung nach Anspruch 6, **dadurch gekennzeichnet, dass** das mittels des reflektierten und/oder gestreuten Lichts erzeugte Bild spektral aufgelöst bzw. selektiert ist.

8. Vorrichtung nach Anspruch 7, **dadurch gekennzeichnet, dass** das spektral aufgelöste bzw. selektierte Bild durch Punkt- oder Linienscannen gewonnen wird.

9. Vorrichtung nach einem der Ansprüche 1 bis 8, **dadurch gekennzeichnet, dass** ein Bildsensor (50, 51, 52) zur Erzeugung eines Fluoreszenzbilds vorgesehen ist.

10. Vorrichtung nach einem der Ansprüche 1 bis 9, **dadurch gekennzeichnet, dass** die 3D-Daten zur Längen-, Flächen- und/oder Volumenmessung auswertbar sind.

11. Vorrichtung nach einem der Ansprüche 1 bis 10, **dadurch gekennzeichnet, dass** aus den 3D-Daten ein Stereobild synthetisiert wird.

12. Vorrichtung nach einem der Ansprüche 1 bis 11, **dadurch gekennzeichnet, dass** die Steuerungs- und Auswertemittel zur Erzeugung einer synoptischen Darstellung der von dem phasenempfindlichen und mindestens einem weiteren Bildsensor gelieferten Daten ausgebildet sind.

13. Vorrichtung nach einem der Ansprüche 1 bis 12, **dadurch gekennzeichnet, dass** Lichterzeugungsmittel zur Erzeugung mindestens einer weiteren Beleuchtungsstrahlung vorgesehen sind.

14. Verfahren zur endoskopischen 3D-Datenerfassung, mit den folgenden Schritten:
- Erzeugung mindestens einer modulierten Messstrahlung,
- Weiterleitung der Messstrahlung auf einen zu beobachtenden Bereich durch mindestens eine endoskopisch einsetzbare Beleuchtungsoptik,
- Abbildung einer Signalstrahlung aus dem zu beobachtenden Bereich mindestens auf einen phasenempfindlichen Bildsensor (50), der eine Mehrzahl von Pixeln aufweist, durch mindestens eine endoskopisch einsetzbare Abbildungsoptik,
- wobei die Beleuchtungsoptik und die Abbildungsoptik in einem endoskopisch einsetzbaren Schaft (29) angeordnet sind, und
- Erzeugung von 3D-Daten durch Auswertung der von dem phasenempfindlichen Bildsensor gelieferten Daten,
- wobei mindestens ein weiterer Bildsensor (51, 52) und ein Strahlteiler (41) zur Aufteilung der empfangenen Strahlung auf verschiedene Sensoren und/oder ein optischer Umschalter zum Umschalten zwischen verschiedenen Sensoren vorgesehen sind,
**dadurch gekennzeichnet, dass**
- die Bildsensoren (50, 51, 52) unterschiedliche Sensorgrößen und/oder Sensorformate aufweisen und dass
- mindestens eine Anpassungsoptik (42) vorgesehen ist zur Anpassung der Gesichtsfelder der Bildsensoren (50, 51, 52), so dass das auf einem Bildsensor (50, 51, 52) erzeugte Bild das gleiche Gesichtsfeld abbildet wie auf einem oder mehreren anderen Bildsensoren (50, 51, 52).

## Claims

1. Device for endoscopic 3D data acquisition, comprising:
- light-generation means for generating at least one modulated measurement radiation,
- light-transmission means for transmitting the measurement radiation to a region to be observed, wherein the light-transmission means comprise an illumination optical unit which can be used endoscopically,
- light-imaging means for imaging signal radiation from the region to be observed, at least on one phase-sensitive image sensor (50), which comprises a plurality of pixels, wherein the light-imaging means comprise an imaging optical unit which can be used endoscopically,
- wherein the illumination optical unit and the imaging optical unit are arranged in a shaft (29) which can be used endoscopically, and
- control and evaluation means for controlling the light-generation means, for actuating the phase-sensitive image sensor and for evaluating the data supplied by the phase-sensitive image sensor for generating 3D data,
- wherein at least one further image sensor (51, 52) is provided and the light-imaging means comprise a beam splitter (41) for dividing the received radiation among different sensors, and/or an optical switching means for switching between different sensors,
**characterized in that**
- the image sensors (50, 51, 52) have different sensor dimensions and/or sensor formats and **in that**
- at least one adaptation optical unit (42) is provided for adapting the fields of view of the image sensors (50, 51, 52) such that the image generated on one image sensor (50, 51, 52) images the same field of view as on one or more other image sensors (50, 51, 52).

2. Device according to Claim 1, **characterized in that** a camera arrangement (30) is provided, which contains at least the phase-sensitive image sensor (50) and which is detachably connected to the shaft (29) which can be used endoscopically.

3. Device according to Claim 2, **characterized in that** the illumination optical unit, the imaging optical unit and the common shaft (29) which can be used endoscopically are parts of a standard endoscope (20).

4. Device according to one of Claims 1 to 3, **characterized in that** at least the imaging optical unit, the phase-sensitive image sensor (50), at least one further image sensor (51, 52) and at least one adaptation optical unit (42) are arranged within the shaft (29) which can be used endoscopically, in the vicinity of the distal end thereof.

5. Device according to one of Claims 1 to 4, **characterized in that** means for coherence reduction are provided.

6. Device according to one of Claims 1 to 5, **characterized in that** an image sensor for generating an image by means of the reflected and/or scattered light is provided.

7. Device according to Claim 6, **characterized in that** the image generated by means of the reflected and/or scattered light is spectrally resolved or selected.

8. Device according to Claim 7, **characterized in that** the spectrally resolved or selected image is obtained by point scanning or line scanning.

9. Device according to one of Claims 1 to 8, **characterized in that** an image sensor (50, 51, 52) for generating a fluorescence image is provided.

10. Device according to one of Claims 1 to 9, **characterized in that** the 3D data can be evaluated for a length, area and/or volume measurement.

11. Device according to one of Claims 1 to 10, **characterized in that** a stereo image is synthesized from the 3D data.

12. Device according to one of Claims 1 to 11, **characterized in that** the control and evaluation means are embodied to generate a synoptic display of the data supplied by the phase-sensitive image sensor and at least one further image sensor.

13. Device according to one of Claims 1 to 12, **characterized in that** light-generation means are provided for generating at least one further illumination radiation.

14. Method for endoscopic 3D data acquisition, comprising the following steps:
- generating at least one modulated measurement radiation,
- transmitting the measurement radiation to a region to be observed by means of at least one illumination optical unit which can be used endoscopically,
- imaging signal radiation from the region to be observed, at least on one phase-sensitive image sensor (50), which comprises a plurality of pixels, by means of at least one imaging optical unit which can be used endoscopically,
- wherein the illumination optical unit and the imaging optical unit are arranged in a shaft (29) which can be used endoscopically, and
- generating 3D data by evaluating the data supplied by the phase-sensitive image sensor,
- wherein at least one further image sensor (51, 52) and one beam splitter (41) for dividing the received radiation among different sensors, and/or an optical switching means for switching between different sensors are provided, **characterized in that**
- the image sensors (50, 51, 52) have different sensor dimensions and/or sensor formats and **in that**
- at least one adaptation optical unit (42) is provided for adapting the fields of view of the image sensors (50, 51, 52) such that the image generated on one image sensor (50, 51, 52) images the same field of view as on one or more other image sensors (50, 51, 52).

## Revendications

1. Dispositif de détection endoscopique de données 3D comprenant :
- des moyens générateurs de lumière destinés à générer au moins un rayonnement de mesure modulé,
- des moyens de retransmission de lumière destinés à retransmettre le rayonnement de mesure sur une zone à observer, les moyens de retransmission de lumière comprenant une optique d'éclairage utilisable de manière endoscopique,
- des moyens de formation d'image de lumière destinés à former l'image d'un rayonnement de signal provenant de la zone à observer sur au moins un capteur d'image sensible à la phase (50) présentant une pluralité de pixels, les moyens de formation d'image de lumière comprenant une optique de formation d'image utilisable de manière endoscopique,
- dans lequel l'optique d'éclairage et l'optique de formation d'image sont disposées dans une tige utilisable de manière endoscopique (29), et
- des moyens de commande et d'évaluation destinés à commander le moyen générateur de lumière pour commander le capteur d'image sensible à la phase et évaluer les données délivrées par le capteur d'image sensible à la phase afin de générer des données 3D,
- dans lequel il est prévu au moins un capteur d'image supplémentaire (51, 52) et les moyens de formation d'image de lumière comprennent un diviseur de faisceaux (41) destiné à diviser le rayonnement reçu sur des capteurs différents et/ou un dispositif de permutation destiné à effectuer une permutation entre différents capteurs,
**caractérisé en ce que**
- les capteurs d'image (50, 51, 52) présentent des tailles de capteurs et/ou des formats de capteurs différents et **en ce que**
- il est prévu au moins une optique d'adaptation (42) destinée à adapter les champs visuels des capteurs d'image (50, 51, 52) de manière à ce que l'image générée sur un capteur d'image (50, 51, 52) forme une image du même champ visuel que sur un ou plusieurs autres capteurs d'image (50, 51, 52).

2. Dispositif selon la revendication 1, **caractérisé en ce qu'**il est prévu un système de caméra (30) comportant au moins le capteur d'image sensible à la phase (50) et connecté de manière amovible à la tige utilisable de manière endoscopique (29).

3. Dispositif selon la revendication 2, **caractérisé en ce que** l'optique d'éclairage, l'optique de formation d'image et la tige commune utilisable de manière endoscopique (29) font partie d'un endoscope standard (20).

4. Dispositif selon l'une quelconque des revendications 1 à 3, **caractérisé en ce qu'**au moins l'optique de formation d'image, le capteur d'image sensible à la phase (50), au moins un capteur d'image supplémentaire (51, 52) et au moins une optique d'adaptation (42) sont disposés à l'intérieur de la tige utilisable de manière endoscopique (29) à proximité de son extrémité distale.

5. Dispositif selon l'une quelconque des revendications 1 à 4, **caractérisé en ce qu'**il est prévu des moyens destinés à réduire la cohérence.

6. Dispositif selon l'une quelconque des revendications 1 à 5, **caractérisé en ce qu'**il est prévu un capteur d'image destiné à générer une image au moyen de la lumière réfléchie et/ou diffusée.

7. Dispositif selon la revendication 6, **caractérisé en ce que** l'image générée au moyen de la lumière réfléchie et/ou diffusée est résolue ou sélectionnée spectralement.

8. Dispositif selon la revendication 7, **caractérisé en ce que** l'image résolue ou sélectionnée spectralement est obtenue par balayage ponctuel ou linéaire.

9. Dispositif selon l'une quelconque des revendications 1 à 8, **caractérisé en ce qu'**il est prévu un capteur d'image (50, 51, 52) destiné à générer une image de fluorescence.

10. Dispositif selon l'une quelconque des revendications 1 à 9, **caractérisé en ce que** les données 3D peuvent être évaluées de manière à mesurer des longueurs, des surfaces et/ou des volumes.

11. Dispositif selon l'une quelconque des revendications 1 à 10, **caractérisé en ce qu'**une image stéréoscopique est synthétisée à partir des données 3D.

12. Dispositif selon l'une quelconque des revendications 1 à 11, **caractérisé en ce que** les moyens de commande et d'évaluation sont conçus pour générer une représentation synoptique des données délivrées par le capteur d'image sensible à la phase et par l'au moins un capteur d'image supplémentaire.

13. Dispositif selon l'une quelconque des revendications 1 à 12, **caractérisé en ce qu'**il est prévu des moyens générateurs de lumière destinés à générer au moins un rayonnement d'éclairage supplémentaire.

14. Procédé de détection endoscopique de données 3D, comprenant les étapes consistant à :
- générer au moins un rayonnement de mesure modulé,
- retransmettre le rayonnement de mesure sur une zone à observer par l'intermédiaire d'au moins une optique d'éclairage utilisable de manière endoscopique,
- former l'image d'un rayonnement de signal provenant de la zone à observer sur au moins un capteur d'image sensible à la phase (50) comportant une pluralité de pixels, par l'intermédiaire d'au moins une optique de formation d'image utilisable de manière endoscopique,
- dans lequel l'optique d'éclairage et l'optique de formation d'image sont disposées dans une tige utilisable de manière endoscopique (29), et
- générer des données 3D par évaluation des données délivrées par le capteur d'image sensible à la phase,
- dans lequel il est prévu au moins un capteur d'image supplémentaire (51, 52) et un diviseur de faisceaux (41) destiné à diviser le rayonnement reçu sur des capteurs différents et/ou un dispositif de permutation optique destiné à effectuer une permutation entre différents capteurs,
**caractérisé en ce que**
- les capteurs d'image (50, 51, 52) présentent des tailles de capteurs et/ou des formats de capteurs différents et **en ce que**
- il est prévu au moins une optique d'adaptation (42) destinée à adapter le champ visuel des capteurs d'image (50, 51, 52) de manière à ce que l'image générée sur un capteur d'image (50, 51, 52) forme une image du même champ visuel que sur un ou plusieurs autres capteurs d'image (50, 51, 52).
